# EUROPEAN PATENT APPLICATION

(11) **EP 3 696 820 A1**
(43) Date of publication of application: **19.08.2020**
(21) Application number: 19157747.7
(22) Date of filing: 18.02.2019
(51) Int. Cl.: G16H 30/40, G06T 7/00, G16H 30/20, G16H 10/40

(54) **INTEGRATED MACRO IMAGE PREVIEW**

(71) Applicant: Koninklijke Philips N.V., 5656 AG Eindhoven (NL)
(72) Inventor: VERHAGEN, Dirk, 5656 AE Eindhoven (NL)
(74) Representative: de Haan, Poul Erik

(57) **Abstract**

The present invention relates to the field of digital pathology. In order to improve the diagnostic workflow, a data processing apparatus is provided for handling images of a sample. The data processing apparatus comprises a data interface, a processing unit, a display unit, and a user interface. The data interface is configured to receive a digital slide of the sample. The digital slide comprises first image data representing an initial macro snapshot of a slide glass on which the sample is provided, wherein the initial macro snapshot comprises at least one electronically identified region representing at least one area containing tissue and second image data representing a whole slide image of the slide glass in form of an image of the at least one electronically identified region in the initial macro snapshot at a predetermined magnification. The processing unit is configured to modify the digital slide displayed on the display unit based on a user operation received from the user interface. the processing unit is configured to display the second image data representing the whole slide image on the display unit in response to a user-initiated zoom-in operation on the first image data representing the initial macro snapshot.

## Description

### FIELD OF THE INVENTION

The present invention relates to the field of digital pathology, and in particular to a data processing apparatus and to a method for handling images of a sample. Furthermore, the present invention relates to a computer program element as well as to a computer-readable medium.

### BACKGROUND OF THE INVENTION

Whole slide scanning generates digital representations of glass slides that with the proper tools can be navigated in an interactive manner. Whole slide scanners capture images of tissue sections tile by tile or in a line-scanning fashion. The multiple images are captured and digitally assembled or stitched to generate a digital image of the entire slide. In order to reproduce the magnifications necessary for many diagnostic and research applications, the glass slide must be captured at sufficiently high resolution and with adequate color depth. A single whole slide image in practice often exceeds 1 GB in size. A method commonly used in whole slide imaging to reduce file size is to discard blank regions of the glass slide altogether. Thus, only a limited area is scanned in high resolution. However, the automatic detection may not identify all tissue on the glass slides. If it is found that there is tissue on the initial macro snapshot or on the glass slide, which cannot be found on the high resolution image, a clinician will need to take appropriate action to ensure the correct diagnosis.

WO 2018/065434 A1 describes that it is possible to toggle between the initial macro snapshot and the whole slide image. A user would then see either the photo of the tissue region (the initial macro snapshot), or the high-resolution scan. However, a user still has to manually apply this toggle to switch between the initial macro snapshot and the whole slide image.

### SUMMARY OF THE INVENTION

There may be a need to improve the diagnostic workflow.

The object of the present invention is solved by the subject-matter of the independent claims, wherein further embodiments are incorporated in the dependent claims. It should be noted that the following described aspects of the invention apply also for the data processing apparatus, for the method for handling images of a sample, for the computer program element, and for the computer-readable medium.

A first aspect of the invention relates to a data processing apparatus for handling images of a sample. The data processing apparatus comprises a data interface, a processing unit, a display unit, and a user interface. The data interface is configured to receive a digital slide of the sample. The digital slide comprises first image data representing an initial macro snapshot of a slide glass on which the sample is provided, wherein the initial macro snapshot comprises at least one electronically identified region with markings representing at least one area containing tissue that is available in a whole slide image of the slide glass, and second image data representing a whole slide image of the slide glass in form of an image of the at least one electronically identified region in the initial macro snapshot at a predetermined magnification. The processing unit is configured to modify the digital slide displayed on the display unit based on a user operation received from the user interface. The processing unit is configured to display the second image data representing the whole slide image on the display unit in response to a user-initiated zoom-in operation on the first image data representing the initial macro snapshot.

Digital pathology requires digitalizing of tissue slides e.g. at ×20, ×40, ×60 or even higher magnification, resulting in a file size of more than 1 GB. A method commonly used in the whole slide imaging to reduce file size is to discard blank regions of the slide. This may be done by identifying regions in the initial macro snapshot that need to be scanned. The whole slide scanning systems typically detect these regions on the glass slide automatically. However, it cannot be ensured that all the tissue areas on the glass slide can be identified. Hence, there are multiple points in the workflow where it is advisable to check whether all tissue has been scanned. It is often possible to toggle between the initial macro image and the whole slide image for checking whether all tissue has been scanned. A user would then see either the photo of the tissue region or the high resolution scan, and can compare the two. However, as the user is required to manually apply this toggle, he/she may have a higher chance of forgetting or skipping this operation and thus may have an incorrect interpretation of the digital slide, thereby resulting in an incorrect diagnosis.

In order to improve the diagnostic workflow and to reduce the risk of an incorrect diagnosis, a data processing apparatus is provided, which allows a user to switch the displayed digital slide from the initial macro snapshot to the high-resolution whole slide image as part of zooming in of the image instead of using a manual toggle through the viewport on the display to choose which image to display. If the first image data representing the initial macro snapshot are displayed on the display unit, the processing unit is configured to display the second image data representing the whole slide image on the display unit, in response to a zoom-in operation received from the user interface. For example, the data processing apparatus may allow a user to handle images of a sample in the following workflow: when a user opens a digital slide of the sample, the initial macro snapshot is shown without a whole slide image overlay. This may allow the user immediately and almost subconsciously check whether all tissue area on the initial macro snapshot is correctly detected. If the user performs a zoom-in operation, the initial macro snapshot is automatically switched to a whole slide image for further analysis. As the zoom-in operation is an action a user does naturally in high-resolution image and as such has a much lower chance of being skipped, it may be ensured that the user, e.g. a clinician, makes the correct diagnosis, based on all available tissue. Furthermore, as this operation is the same as the main operation to view the whole slide image, the user is not required to perform a different, additional operation, such as a manual toggle through the viewport on the display to choose the whole slide image to display. This may streamline work processes and improve the diagnostic workflow to handle images of a sample.

The term "sample" relates to a (small) portion of the biological material, e.g. a thin slice of the biological material, such as tissue, which are obtained by sectioning e.g. paraffin-embedded pathological specimen (after e.g. chemical fixation, processing and embedding procedures) into thin slices. The thickness of a slice may relate to an order of a few micrometers. For example, the thickness of a sample slice may be 2 to 4 micrometers. Depending on the applications, a sample slice may also have a thickness ranging from 0.5 to 50 micrometers. For example, several sample slices are cut from the object one after the other.

The term "slide glass" as used herein may refer to a physical slide glass, onto which sample slices are then mounted onto (glass) slides for precise visualization and/or for image acquisition.

The term "digital slide of the sample" as used herein may refer to a digital representation of a sample slice on a sample slide - hence, also referred to as a digital slide, a digitized glass slide or a virtual slide. The digital slide may comprise low-resolution image data of the sample captured with a low-resolution camera. The digital slide may comprise high-resolution image data of the sample acquired using e.g. a whole slide scanner. The high-resolution image data may be stored at multiple resolutions to accommodate a streamlined method for loading images. These images may be embedded within a single file, although this is not a requirement. The digital slide of the sample may also be stored locally or in an image management system (IMS).

The term "initial macro snapshot" as used herein may refer to a single low-resolution image of the slide glass. The initial macro snapshot may also be referred to as a macro image or a thumbnail image. The initial macro snapshot is typically captured with a low-resolution camera representing the entire tissue fit within a ∼1-megapixel frame. On the initial macro snapshot the regions that are electronically identified are clearly indicated.

The term "whole slide image" as used herein may refer to a high-resolution image of the slide glass often exceeding 1 GB in size. The whole slide image is typically captured with a different system such as a high resolution optical system, e.g. a scanning system, and typically part of a multi resolution image structure. Whole slide scanners may capture images of tissue sections tile by tile or in a line-scanning fashion. The multiple images, either tiles or lines, are captured and digitally assembled to generate a digital image of the entire slide, i.e. the whole slide image. The scan of an entire slide may introduce a potential burden for storage and scan times. In order to reduce file size and scan times, a method commonly used in whole slide imaging is to identify regions in the initial macro snapshot that need to be scanned and blank regions in the initial macro snapshot that need to be discarded.

The term "electronically identified regions" as used herein may refer to the regions that are identified by technical means, such as a whole slide scanner. In other words, the regions are not manually identified by a person.

According to an embodiment of the invention, the processing unit is configured to display the first image data representing the initial macro snapshot overlaid with the second image data representing a whole slide image in response to the user-initiated zoom-in operation on the first image data representing the initial macro snapshot.

In other words, the zoom-in operation switches the displayed digital slide from an initial macro snapshot without a whole slide image overlay to an initial macro snapshot with a whole slide image overlay. As both images are on the same scale, the user may immediately detect the missing tissue by comparing the whole slide image overlay with the indicated electronically identified regions on the initial macro snapshot.

According to an embodiment of the invention, the processing unit is configured to display the first image data representing the initial macro snapshot, when the second image data representing the whole slide image is displayed and zoomed out to such an extent that the displayed second image data representing the whole slide image fit a viewport of the display unit.

In other words, when zooming out, the user zooms out no further than when the whole slide image fits in the view port. As soon as that state is reached and the user zooms out further, the whole slide image is automatically switched back to the initial macro snapshot. This may ensure that the user can easily find the initial macro snapshot by simply zooming out without the need to manually toggle to the initial macro snapshot. Using the zoom-in and zoom-out operation to switch between the initial macro snapshot and the whole slide image thus facilitates the workflow to handle the images of a sample.

According to an embodiment of the invention, the processing unit is configured to display the first image data representing the initial macro snapshot on the display unit in response to a user operation of opening a digital slide of the sample.

In other words, the user may start in the initial macro snapshot and can thus immediately detect the missing tissue by checking the indicated electronically identified regions and whether all relevant tissue is comprised in these regions. In particular, by doing this it may be ensured that there is always a subconscious check whether all tissue is scanned. There are two options to display the initial macro snapshot in response to the operation of opening the digital slide of the sample. The first option is to display the initial macro snapshot every time the user open the digital slide of the sample. The second option is to display the initial macro snapshot the first time the user opens the image of the sample. If the user opens the image again, it could open in the same area as where they were looking. The data processing apparatus may allow a user to specify one of these options e.g. via a dialog box.

According to an embodiment of the invention, the processing unit is configured to display a previously displayed area of the second image data representing an area, which was displayed when the second image data of the sample was last closed, in response to a user operation of re-opening the image of the sample.

Thus, the user may have a second option to display the initial macro snapshot the first time the user opens the image of the sample. If the user opens the image again, it could open in the same area as where they were looking.

According to an embodiment of the invention, the data processing apparatus further comprises a storage unit adapted for storing the digital slide of the sample.

A second aspect of the invention relates to a method for handling images of a sample. The method comprises the step of receiving a digital slide of the sample. The digital slide comprises first image data representing an initial macro snapshot of a slide glass on which the sample is provided, wherein the initial macro snapshot comprises at least one electronically identified region with markings representing at least one area containing tissue that is available in a whole slide image of the slide glass, and second image data representing a whole slide image of the slide glass in form of an image of the at least one electronically identified region in the initial macro snapshot at a predetermined magnification. The second image data representing the whole slide image are displayed in response to a user-initiated zoom-in operation on the first image data representing the initial macro snapshot.

Thus, instead of using a manual toggle to switch the digital slide from an initial macro snapshot to a whole slide image, a user switches the displayed digital slide from the initial macro snapshot to the whole slide image as part of zooming in of the image. This is the action a user does naturally in high-resolution whole slide image and as such has a lower chance of being skipped. The use of the zoom-in operation to switch the displayed digital slide from the initial macro snapshot to the whole slide image may streamline work processes and improve workflow to handle images of a sample.

According to an embodiment of the invention, the method further comprises displaying the first image data representing the initial macro snapshot overlaid with the second image data representing a whole slide image in response to the user-initiated zoom-in operation on the first image data representing the initial macro snapshot.

This may allow the user to immediately check all tissue area was correctly digitized and the scanner detected all tissue by checking the marked electronically identified regions on the initial macro snapshot against the whole slide image.

According to an embodiment of the invention, the method further comprises the step of displaying the first image data representing the initial macro snapshot, when the second image data representing the whole slide image is displayed and zoomed out to such an extent that the displayed second image data representing the whole slide image fit a viewport of the display unit.

In other words, when zooming out, a user can zoom out no further than when the whole slide image fits in the viewport. As soon as that state is reached and the user zooms out further, the whole slide image is automatically switched back to the initial macro snapshot. This ensures that the user can easily find the initial macro snapshot by simply zooming out. Thus, the user can switch between the whole slide image and the initial macro snapshot as part of zooming in and zooming out of the image. With the zoom-in and zoom-out operations, there is less chance of skipping the step of checking the missing tissue.

According to an embodiment of the invention, the method further comprises the step of displaying the first image data representing the initial macro snapshot in response to a user operation of opening the digital slide of the sample.

This may allow the user to immediately check whether all tissue is available in the whole slide image when the user opens the image of the sample and the initial macro snapshot is displayed. This may further reduce the chance of forgetting or skipping the step of determining whether all tissue area was correctly digitized.

According to an embodiment of the invention, the method further comprises the step of displaying a previously displayed area of the second image data representing an area of the second image data, which was displayed when the second image data of the sample was last closed, in response to a user operation of re-opening the digital slide of the sample.

This may allow the user to choose an option to display the initial macro snapshot the first time the user opens the image of the sample. If the user opens the image again, it could open in the same area as where they were looking.

A third aspect of the invention relates to a computer program element for controlling an apparatus as described above and below, which, when being executed by a processing unit, is adapted to perform the method steps as described above and below.

A fourth aspect of the invention relates to a computer readable medium having stored the above-mentioned program element.

According to another aspect of the present invention, a data processing apparatus is presented to allow a user to handle images of a sample, in particular to check whether all tissue is scanned on a glass slide before performing diagnosis on a digital image, in a more simplified and streamlined way. A commonly used method is to toggle between the initial macro snapshot and the whole slide image to check whether all tissue is scanned to ensure the correct diagnosis. A user would then see either the photo of the tissue region, or the high-resolution scan. However, a user still has to manually apply this toggle. As a user's primary task is making a diagnosis they typically start with the whole slide image overlay on. The solution provides a simple possibility to solve this problem. In particular, when a user opens an image using the data processing apparatus, the initial macro snapshot is shown without a whole slide image overlay. This means the user can immediately and almost subconsciously check whether all tissue area on the initial macro snapshot is also detected, e.g. the tissue area having a rectangle around it. The user will naturally use the mouse wheel to zoom in on certain regions. As they do so, the display automatically switches to the high resolution image for a seamless experience. So instead of using a manual toggle to switch between the whole slide image and the macro, a user switches between the macro and the whole slide image as part of zooming in and zooming out of the image. These are actions a user does naturally in high resolution image and as such have a much lower chance of being skipped. When zooming out, the displayed image zooms out no further than when the whole slide image fits in the viewport. As soon as that state is reached and the users zooms out further, the displayed image automatically switches back to the initial macro snapshot. This ensures users can easily find the initial macro snapshot by simply zooming out. This will feel more natural than mode switching. It is possible to extend this by only showing the initial macro snapshot the first time the user opens an image. If a user opens the image again, it could open in the same area as where they were looking.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1A shows a photo of an initial macro snapshot of a physical glass slide.
Fig. 1B shows a photo of an initial macro snapshot overlaid with a whole slide image.
Fig. 2 shows an example of a workflow to toggle between an initial macro snapshot and a whole slide image.
Fig. 3 shows a further example of a workflow to toggle between an initial macro snapshot and a whole slide image.
Fig. 4 shows a schematic drawing of a data processing apparatus for handling images of a sample according to an exemplary embodiment of the present disclosure.
Fig. 5 shows a workflow to handle images of a sample using a data processing apparatus according to an exemplary embodiment of the present disclosure.
Fig. 6 shows another workflow to handle images of a sample using a data processing apparatus according to an exemplary embodiment of the present disclosure.
Fig. 7 shows a flow diagram of a method of handling images of a sample according to an exemplary embodiment of the present disclosure.

The figures are schematic and not at scale and are not used for graphical purposes.

### DETAILED DESCRIPTION OF EMBODIMENTS

In general, any system, which automatically images part of a physical artefact, often has to indicate which part of the physical artefact was imaged. A user then has to be able to check whether all the areas on the physical artefact, which were intended to be imaged, are also imaged. For the field of pathology, this is a particular problem when scanning tissue, i.e. the part that needs to be imaged on glass slides, i.e. the physical artefact. Whole slide imaging systems for pathology are used to convert glass slides into very high resolution digital images. They can then base a diagnosis, or a second opinion on these digital images, instead of on the glass slides. This eliminates the need for physical logistics. These whole slide scanning systems typically detect the tissue on the glass slide automatically. After tissue detection, the areas of the glass slide containing tissue are scanned in high resolution.

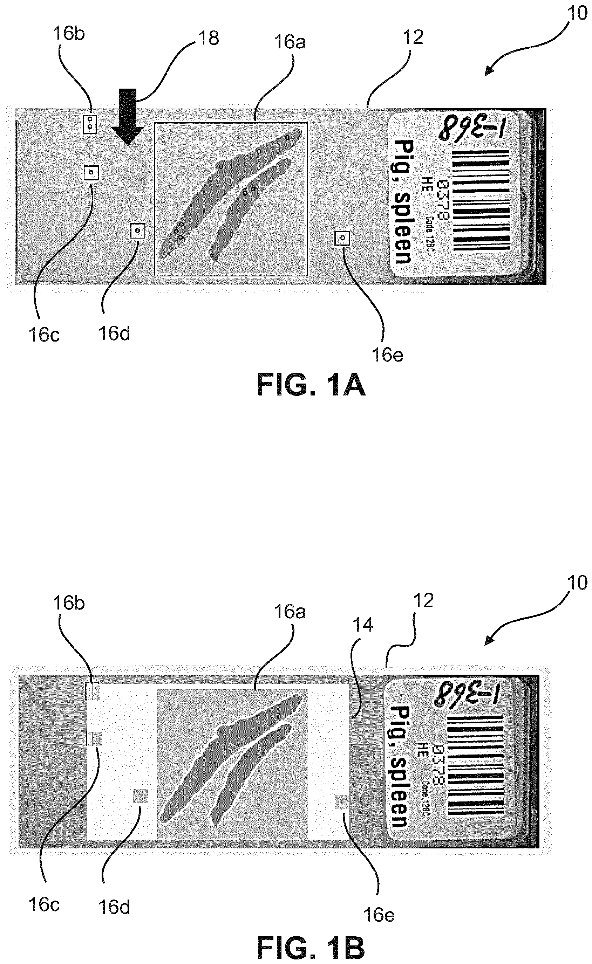

Figures 1A and 1B illustrate an initial macro snapshot 12 and a whole slide image 14 of a digital slide 10, namely a digital representation of a physical glass slide. In Figure 1A, the initial macro snapshot 12 is illustrated, which has five marked regions 16a-16e representing areas containing tissue, each being surrounded by a rectangle. The circles inside the marked regions 16a-16e represent small patches of tissue. These marked regions 16a-16e were automatically identified e.g. by a whole slide scanning system. Only these marked regions 16a-16e on the initial macro snapshot 10 are scanned in high resolution to reduce file size. It is also noted that such automatic detection of areas containing tissue may not be accurate. For example, there is tissue at an arrow 18 not found by the whole slide scanning system. Thus, the tissue at the arrow 18 is not available in the high-resolution whole slide image. This may be seen clearly in the whole slide image 14 as illustrated in Figure IB. In Figure 1B, the initial macro snapshot 12 is displayed with the whole slide image 14 overlay. The marked regions 16a-16e in the initial macro snapshot 12 as illustrated in Figure 1A are all available in the whole slide image 14. On the other hand, the tissue at the arrow 18 is not scanned. It is also occluded by the overlay and thus no longer visible. To determine whether all tissue area was correctly digitized and the scanner detected all tissue, the high-resolution whole slide image can be checked either against the glass slide, or against a photo of the glass slide, i.e. the initial macro snapshot. If it is found that there is tissue on the initial macro snapshot or on the glass slide, which cannot be found on the high-resolution image, a clinician will need to take appropriate action to ensure the correct diagnosis.

Typically, software enables this by making the photo of the slide available in some separate part of the software, and issuing a warning in the manual to always check your high-resolution section against this photo. However, constant reminders may be seen as an annoyance and will over time, be ignored. Therefore, having the initial macro snapshot of a glass slide in separate windows or parts of the interface is not optimal. Another method is to toggle between the initial macro snapshot and the whole slide image. A user would then see either the photo of the tissue region, or the high-resolution scan. However, a user still has to manually apply this toggle.

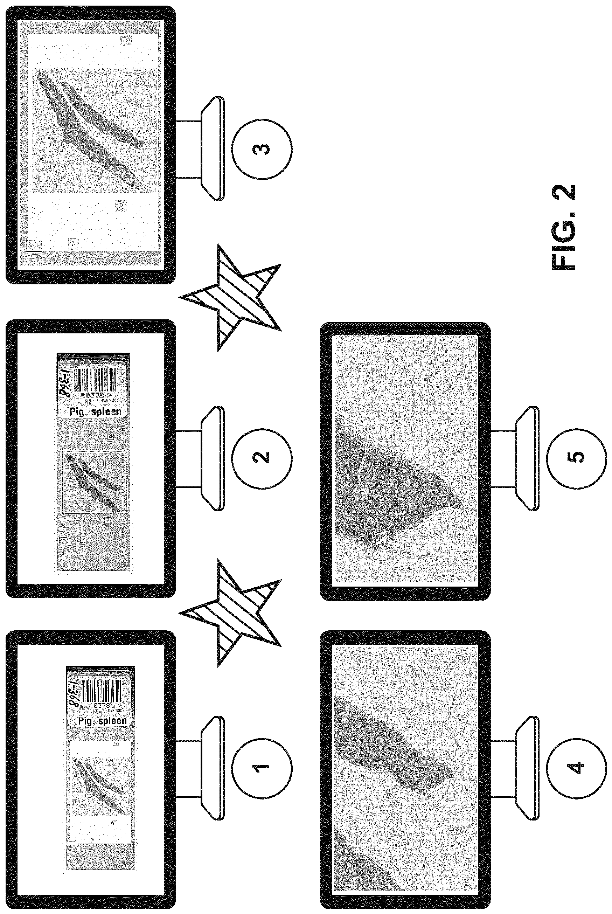

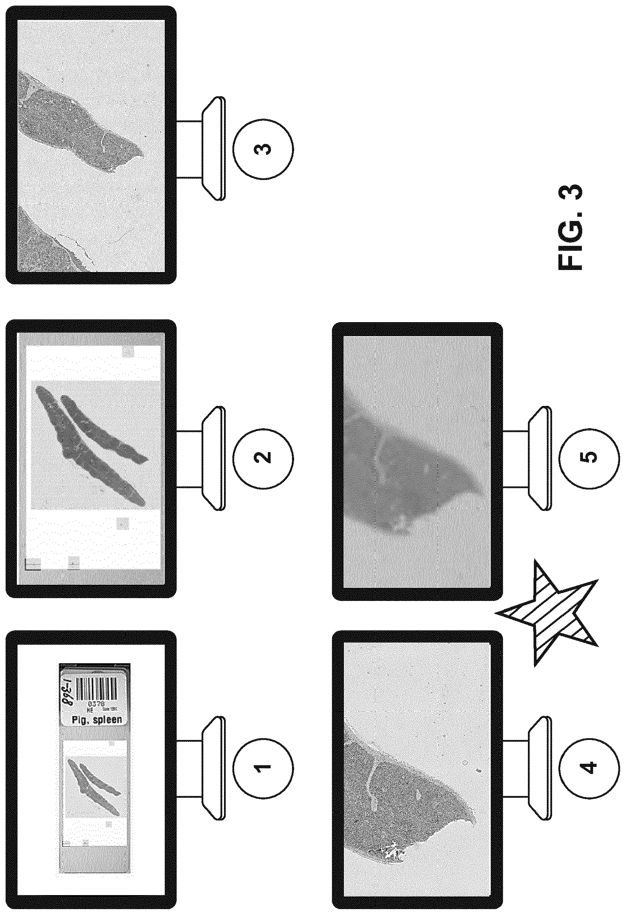

The following examples as shown in Figures 2 and 3 illustrate the variety of workflows to toggle between the initial macro snapshot and the whole slide image. The workflow in Figure 2 is as follows: (1) A user would start with the whole slide image projected on the initial macro snapshot; (2) The user would manually toggle to the initial macro snapshot and notice the missing tissue; (3) The user would than toggle back and find a region of interest; and (4-5) The user would continue zooming in to the region of interest.

Figure 3 illustrate another example of a workflow to toggle between the initial macro snapshot and the whole slide image. The workflow is as follows: (1) A user would start with the whole slide image projected on the initial macro snapshot; (2-4) A user would continue zooming in to the region of interest; and (5) A user would manually toggle to the initial macro snapshot and miss that not all tissue is available in high resolution.

In both scenarios as illustrated in Figures 2 and 3, a manual action beyond the primary task, namely finding and analyzing regions of interest, is needed, which is easily forgotten or skipped. The manual actions are done at the point of the stars.

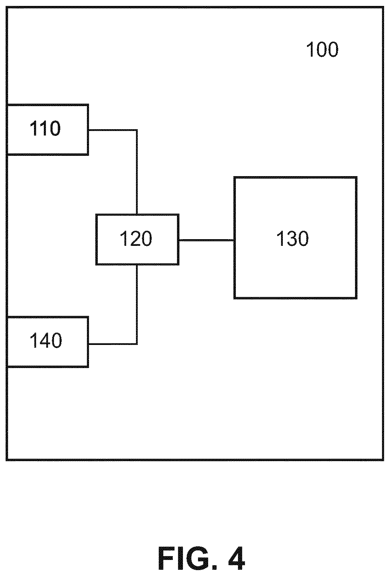

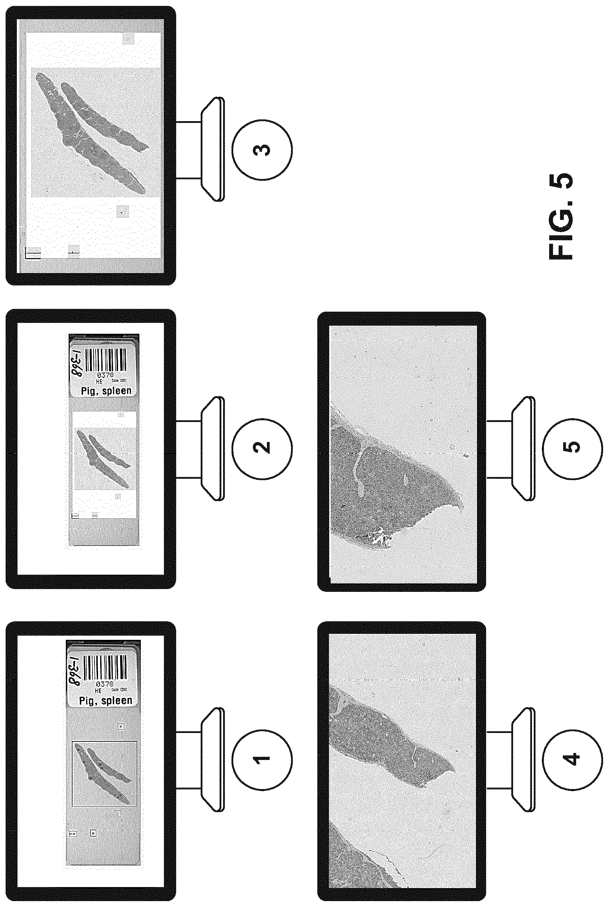

Figure 4 schematically shows a data processing apparatus 100 for handling images of a sample according to an exemplary embodiment of the present disclosure. The data processing apparatus 100 is configured to allow a user to handle images of a sample by following a workflow as illustrated in Figure 5 with improved user experience. The data processing apparatus 100 may be e.g. a personal computer (PC). The data processing apparatus 100 comprises a data interface 110, a processing unit 120, a display unit 130, and a user interface 140.

The data interface 110 may be a universal serial bus (USB) interface, a Bluetooth interface, a wireless network interface, etc. suitable to receive the digital slide of a sample from a whole slide scanner or a digital archive in a cloud-based or local server-based storage.

The processing unit 120 may be e.g. a microprocessor, a microcontroller, a field programmable gate array (FPGA), a central processing unit (CPU), or a digital signal processor (DSP) capable of receiving data, e.g. via a universal service bus (USB), a physical cable, Bluetooth, or another form of data connection and processing the received data.

The display unit 130 may be a display device that uses liquid crystal, electroluminescence (EL), a cathode ray tube (CRT), or the like. The display unit 16 and the data processing apparatus 100 may be formed integrally or separately.

The user interface 140 may be e.g. a pointing device, a keyboard, a touch panel, or another operation apparatus. The user interface 18 in form of a touch panel may also be integrated with the display unit 130.

The data processing apparatus 100 may comprise an optional storage unit (not shown), which may be a non-volatile memory, such as a hard disk drive (HDD), a flash memory, and another solid state memory, adapted for storing the digital slide of the sample.

The data interface 110 is configured to receive a digital slide of the sample, e.g. from a whole slide scanner, a digital archive in a cloud-based or local server-based storage. The digital slide comprises first image data representing an initial macro snapshot of a slide glass on which the sample is provided, wherein the initial macro snapshot comprises at least one electronically identified region representing at least one area containing tissue, and second image data representing a whole slide image of the slide glass in form of an image of the at least one electronically identified region in the initial macro snapshot at a predetermined magnification. Examples of an initial macro snapshot and a whole slide images are illustrated in Figures 1A and IB. In Figure 1A, these electronically identified regions 16a-16e are surrounded by rectangles. As only the electronically identified regions 16a-16e in the initial macro snapshot are scanned, the whole slide image is actually a high-resolution image of the electronically identified regions 16a-16e in the initial macro snapshot at a predetermined magnification. Scanning may occur at multiple magnifications. For example, scanning at ×20 predetermined magnification may be acceptable for standard viewing and interpretation. For certain applications, such as digitization of in situ hybridization slides, images may be acquired at ×40 predetermined magnification to resolve information that may be separated by distances less than 0.5 µm.

The processing unit 120 is configured to modify the digital slide displayed on the display unit 130 based on a user operation received from the user interface 140. The processing unit 120 is configured to display the second image data representing the whole slide image on the display unit in response to a user-initiated zoom-in operation on the first image data representing the initial macro snapshot. In other words, if the first image data representing the initial macro snapshot are displayed on the display unit, the processing unit 120 is configured to display the second image data representing the whole slide image on the display unit 130, in response to a zoom-in operation received from the user interface 140.

Figure 5 illustrates an example of a workflow to handle images of the sample using the data processing apparatus 100. The workflow is as follows: (1) A user starts in the initial macro snapshot of a sample and can immediately detect the missing tissue; (2) If the user performs a zoom-in operation, a whole slide image projected onto the initial macro snapshot is displayed; (3) If the user continues zooming in, the whole slide image is displayed without any overlay; and (4-5) If the user continues zooming in to a region of interest, the regions of interest will be displayed.

With the use of the data processing apparatus, there is no need to manually toggle between the initial macro snapshot and the whole slide image. The user no longer has to take a conscious action. The operations of opening on the initial macro snapshot, tying its use to zooming in and zooming out, and combining these two images are part of the workflow.

Optionally, the processing unit 120 is configured to display the first image data representing the initial macro snapshot, when the second image data representing the whole slide image is displayed and zoomed out to such an extent that the displayed second image data representing the whole slide image fit a viewport of the display unit 130. In other words, if the second image data representing the whole slide image are displayed, the processing unit 120 is configured to display the first image data representing the initial macro snapshot, when the displayed second image data representing the whole slide image is zoomed out to such an extent that the displayed second image data representing the whole slide image fit a viewport of the display unit 130. In other words, if the user continue zooming out, the high-resolution whole slide image is automatically switched to the initial macro snapshot when the whole slide image no longer fits the viewport of the display unit.

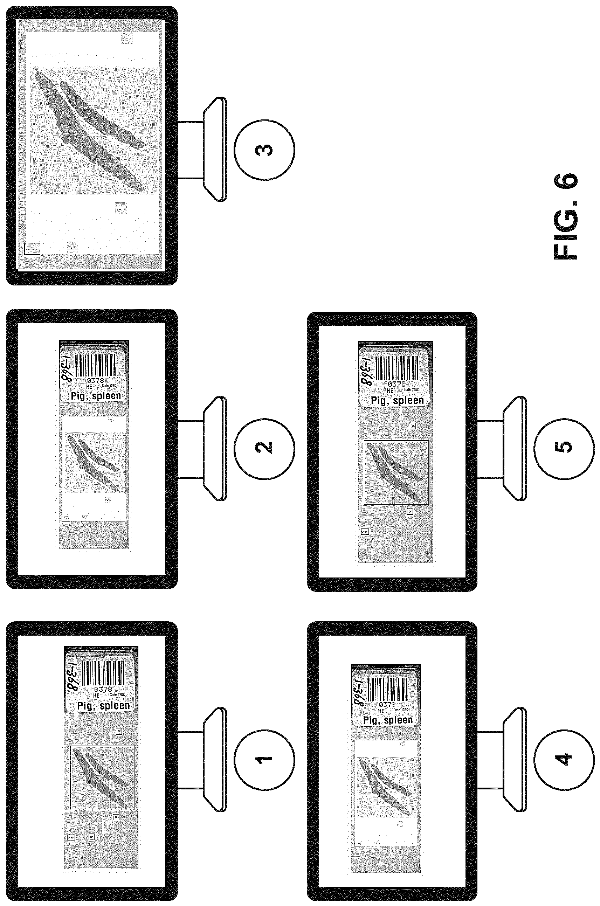

Figure 6 illustrates another example of a workflow to handle images of the sample using the data processing apparatus 100. The workflow is as follows: (1) A user starts in the initial macro snapshot of a sample and can immediately detect the missing tissue; (2) If the user performs a zoom-in operation, a whole slide image projected onto the initial macro snapshot is displayed; (3) If the user continues zooming in, the whole slide image is displayed without any overlay; (4) If the user zooms out, the displayed whole slide image is automatically switched to the initial macro snapshot with a whole slide image overlay, when the displayed whole slide image is zoomed out to such an extent that the displayed whole slide image fits a viewport of the display unit; and (5) If the user continues zooming out, the initial macro snapshot is displayed without any overlay. It is also noted that the workflow step (4) is optional. In other words, if the user zooms out, the displayed whole slide image may be automatically switched to the initial macro snapshot without any overlay as illustrated in workflow step (5), when the displayed whole slide image is zoomed out to such an extent that the displayed whole slide image fits a viewport of the display unit.

Optionally, the processing unit 140 is configured to display the first image data representing the initial macro snapshot on the display unit 130, in response to a user operation of opening the digital slide of the sample received from the user interface 140. There are two options to show the initial macro snapshot in response to an operation of opening the digital slide. One option is to show the initial macro snapshot every time the user opens the digital slide of the sample. The other option is to show the initial macro snapshot the first time the user opens an image. If the user reopens the image, the processing unit 120 is configured to display a previously displayed area of the second image data representing an area, which was displayed when the second image data of the sample was last closed, in response to a user operation of re-opening the digital slide of the sample received from the user interface. A dialog box may be provided and displayed on the display unit to allow the user to choose one of these two options.

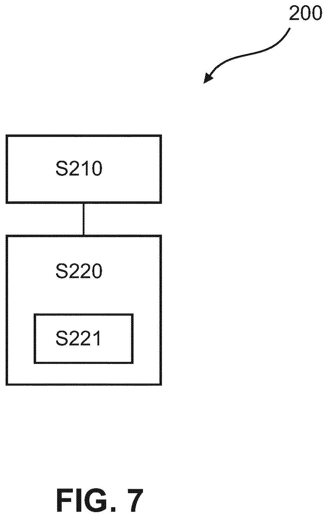

Figure 7 schematically shows a method 200 for handling images of a sample according to an exemplary embodiment of the present disclosure. In step S210, a digital slide of the sample is received. The digital slide comprises first image data representing an initial macro snapshot of a slide glass on which the sample is provided and second image data representing a whole slide image of the slide glass in form of an image of the at least one electronically identified region in the initial macro snapshot at a predetermined magnification. The initial macro snapshot comprises at least one electronically identified region with markings representing at least one area containing tissue that is available in a whole slide image of the slide glass.

The method further comprises the step of displaying S220 the second image data representing the whole slide image in response to a user-initiated zoom-in operation on the first image data representing the initial macro snapshot. In other words, if the first image data representing the initial macro snapshot are displayed, the method further comprises the step of displaying S220 the second image data representing the whole slide image, in response to a zoom-in operation received from the user interface. Optionally, the method further comprises the step of displaying S221 the first image data representing the initial macro snapshot overlaid with the second image data representing a whole slide image in response to the user-initiated zoom-in operation on the first image data representing the initial macro snapshot. In other words, if the first image data representing the initial macro snapshot are displayed on the display unit, the method further comprises the step of displaying S221 the first image data representing the initial macro snapshot overlaid with the second image data representing a whole slide image, in response to the zoom-in operation received from the user interface.

The method shown in Figure 7 can be supplemented, of course, by several additional optional method steps. In particular, the first image data representing the initial macro snapshot is displayed, when the second image data representing the whole slide image is displayed and zoomed out to such an extent that the displayed second image data representing the whole slide image fit a viewport of the display unit. In other words, if the second image data representing the whole slide image are displayed, the first image data representing the initial macro snapshot may be displayed, when the displayed second image data representing the whole slide image is zoomed out to such an extent that the displayed second image data representing the whole slide image fit a viewport of the display unit.

In addition, the first image data representing the initial macro snapshot may be displayed in response to a user operation of opening the digital slide of the sample received from the user interface. Optionally, in response to a user operation of re-opening the digital slide of the sample, a previously displayed area of the second image data may be displayed representing an area of the second image data which was displayed when the second image data of the sample was last closed.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A data processing apparatus (100) for handling images of a sample, comprising:
- a data interface (110);
- a processing unit (120);
- a display unit (130); and
- a user interface (140);
wherein the data interface is configured to receive a digital slide (10) of the sample, wherein the digital slide comprises:
- first image data representing an initial macro snapshot (12) of a slide glass on which the sample is provided, wherein the initial macro snapshot comprises at least one electronically identified region (16a, 16b, 16c, 16d, 16e) with markings representing at least one area containing tissue that is available in a whole slide image of the slide glass; and
- second image data representing a whole slide image (14) of the slide glass in form of an image of the at least one electronically identified region in the initial macro snapshot at a predetermined magnification;
wherein the processing unit is configured to modify the digital slide displayed on the display unit based on a user operation received from the user interface; and
wherein the processing unit is configured to display the second image data representing the whole slide image on the display unit in response to a user-initiated zoom-in operation on the first image data representing the initial macro snapshot.

2. Data processing apparatus according to claim 1,
wherein the processing unit is configured to display the first image data representing the initial macro snapshot overlaid with the second image data representing a whole slide image in response to the user-initiated zoom-in operation on the first image data representing the initial macro snapshot.

3. Data processing apparatus according to claim 1 or 2,
wherein the processing unit is configured to display the first image data representing the initial macro snapshot, when the second image data representing the whole slide image is displayed and zoomed out to such an extent that the displayed second image data representing the whole slide image fit a viewport of the display unit.

4. Data processing apparatus according to any of the preceding claims,
wherein the processing unit is configured to display the first image data representing the initial macro snapshot on the display unit in response to a user operation of opening a digital slide of the sample.

5. Data processing apparatus according to claim 4,
wherein the processing unit is configured to display a previously displayed area of the second image data representing an area, which was displayed when the second image data of the sample was last closed, in response to a user operation of re-opening the digital slide of the sample.

6. Data processing apparatus according to any of the preceding claims, further comprising:
- a storage unit adapted for storing the digital slide of the sample.

7. A method (200) for handling images of a sample, comprising the following steps:
- receiving (S210) a digital slide of the sample, wherein the digital slide comprise first image data representing an initial macro snapshot of a slide glass on which the sample is provided, wherein the initial macro snapshot comprises at least one electronically identified region with markings representing at least one area containing tissue that is available in a whole slide image of the slide glass and second image data representing a whole slide image of the slide glass in form of an image of the at least one electronically identified region in the initial macro snapshot at a predetermined magnification;
- displaying (S220) the second image data representing the whole slide image in response to a user-initiated zoom-in operation on the first image data representing the initial macro snapshot.

8. Method according to claim 7, further comprising:
- displaying (S221) the first image data representing the initial macro snapshot overlaid with the second image data representing a whole slide image in response to the user-initiated zoom-in operation on the first image data representing the initial macro snapshot.

9. Method according to claim 7 or 8, further comprising:
- displaying the first image data representing the initial macro snapshot, when the second image data representing the whole slide image is displayed and zoomed out to such an extent that the displayed second image data representing the whole slide image fit a viewport of the display unit.

10. Method according to any of claims 7 to 9, further comprising:
- displaying the first image data representing the initial macro snapshot in response to a user operation of opening a digital slide of the sample.

11. Method according to claim 10, further comprising:
- displaying a previously displayed area of the second image data representing an area of the second image data which was displayed when the second image data of the sample was last closed, in response to a user operation of re-opening the digital slide of the sample.

12. Computer program element for controlling an apparatus according to one of the claims 1 to 6, which, when being executed by a processing unit, is adapted to perform the method steps of one of the claims 7 to 11.

13. Computer readable medium having stored the program element of claim 12.
